# EUROPEAN PATENT APPLICATION

(11) **EP 4 183 800 A1**
(43) Date of publication of application: **24.05.2023**
(21) Application number: 21209434.6
(22) Date of filing: 19.11.2021
(51) Int. Cl.: C07K 16/10

(54) **NOVEL SARS-COV-2 NEUTRALIZING ANTIBODIES**

(71) Applicant: Medizinische Hochschule Hannover, 30625 Hannover (DE); Twincore, Zentrum für Experimentelle u Klinische Infektionsforschung GmbH, 30625 Hannover (DE)
(72) Inventor: The designation of the inventor has not yet been filed
(74) Representative: Virnekäs, Bernhard

(57) **Abstract**

The present invention relates to novel antibody-based binding composition with specificity for the SARS-CoV-2 spike receptor-binding domain, to a multispecific antibody-based binding composition with specificity for at least two different epitopes on the SARS-CoV-2 spike receptor-binding domain, to a nucleic acid encoding the antibody-based binding composition of the present invention, or the multispecific antibody-based binding composition of the present invention,.to a pharmaceutical composition comprising the antibody-based binding composition of the present invention, the multispecific antibody-based binding composition of the present invention, or the nucleic acid of the present invention, including for use in the prevention or treatment of an infection with SARS-CoV-2.

## Description

### FIELD OF THE INVENTION

The present invention relates to novel antibody-based binding composition with specificity for the SARS-CoV-2 spike receptor-binding domain, to a multispecific antibody-based binding composition with specificity for at least two different epitopes on the SARS-CoV-2 spike receptor-binding domain, to a nucleic acid encoding the antibody-based binding composition of the present invention, or the multispecific antibody-based binding composition of the present invention, to a pharmaceutical composition comprising the antibody-based binding composition of the present invention, the multispecific antibody-based binding composition of the present invention, or the nucleic acid of the present invention, including for use in the prevention or treatment of an infection with SARS-CoV-2.

### BACKGROUND OF THE INVENTION

In the past 20 months, the global community has experienced an unprecedented health, economic and social disaster triggered by the Covid pandemic. In addition to so-called "non-pharmaceutical interventions", such as partial or complete lock-downs, wearing medical masks and adhering to the distancing rules, vaccines have meanwhile become available as "pharmaceutical interventions", an important element in fighting a pandemic. Nevertheless, there is currently a lack of pathogen-specific treatment options, particularly in light of the fact that the percentage of fully vaccinated people has not yet reached satisfactory levels, so that infections of non-vaccinated people as well as break-through infections of vaccinated people still remain a critical problem, particularly in light of the limited number of intensive care units available for severe Covid cases as well as other patients in critical, non-Covid-based situations.

Treatment options for Covid-infected patients with severe symptoms are currently rather limited (see: COVID-19 Treatment Guidelines Panel. Coronavirus Disease 2019 (COVID-19) Treatment Guidelines. National Institutes of Health. Available at https://www.covid19treatmentguidelines.nih.gov/. Accessed November 11, 2021), and mainly include treatment with existing antiviral drugs, such as Remdesivir, albeit with limited success (see WHO Solidarity Trial Consortium; Hongchao Pan et al., Repurposed Antiviral Drugs for Covid-19 - Interim WHO Solidarity Trial Results, N Engl J Med 384 (2021) 497-511), and symptomatic treatment with medicaments approved for inflammatory diseases, such as dexamethasone and other corticosteroids, baricitinib, tofacitinib, tocilizumab or sarilumab.

Thus, there is a high and currently unmet need for pathogen-specific treatment options, and even if the measures currently available ultimately succeed in containing the pandemic, such pathogen-specific treatment options will remain a central component of disease treatment for many years to come.

The development of such treatment approaches is a challenging task.

Antibodies represent a particularly interesting class of pathogen-specific reagents, and the high success rate of the vaccination approaches is strong evidence for the viability of an antibody-based treatment approach. So far, anti-SARS-CoV-2 antibodies have been generated in a number of different ways, including the generation of antibodies from phage-displayed naive libraries (see Bertoglio et al., SARS-CoV-2 neutralizing human recombinant antibodies selected from pre-pandemic healthy donors binding at RBD-ACE2 interface, Nature Communications volume 12, article number: 1577 (2021)) or from genetically engineered mouse platforms (for example, Regeneron's approved antibody Casirivimab).

One particularly promising approach for the identification of antibodies is the isolation of such specific antibodies from convalescent COVID-19 patients, and a number of antibodies identified by that approach have already been described (see, for example, Shi et al. A human neutralizing antibody targets the receptor-binding site of SARS-CoV-2. Nature 584 (2020) 120-124; Jones et al. The neutralizing antibody, LY-CoV555, protects against SARS-CoV-2 infection in nonhuman primates. Sci Transl Med 13, eabf1906 (2021) and Regeneron's approved antibody Imdevimab).

As an additional complication, SARS-CoV-2 may acquire mutations, which may alter infectivity, disease severity and interactions with host immunity, including interactions with host antibodies (see Harvey et al. SARS-CoV-2 variants, spike mutations and immune escape. Nat Rev Microbiol 19 (2021) 409-424). Such altered interaction with host antibodies may explain the substantial degree of break-through infections in fully vaccinated people, as mentioned above. Additionally, the development of escape mutants may severely limit the treatment success, particularly for the treatment with an antibody directed at a single antigenic epitope.

Thus, it has been suggested that treatment with a cocktail of antibodies targeting two or more different epitopes on the spike protein may be suitable for reducing or preventing the successful development of escape mutants under treatment (see Baum, A. et al. Antibody cocktail to SARS-CoV-2 spike protein prevents rapid mutational escape seen with individual antibodies. Science 369 (2020) 1014-1018).

Accordingly, Regeneron obtained FDA approval for a cocktail of the two antibodies Casirivimab & Imdevimab mentioned above. In a phase III trial in high-risk symptomatic outpatients, it could be shown that hospitalization and/or death were reduced by 70 %. In a second phase III trial in non-symptomatic outpatients, it could be shown that in addition to reducing the risk of symptomatic infections, the total number of weeks patients experienced symptoms was reduced to 45 %, and the viral burden was reduced by more than 90 %.

Despite these promising results, the trial results demonstrate that there is still substantial room for additional improvement. Furthermore, in light of the potential development of escape mutants that may render the approved drug cocktail ineffective, the development of back-up candidates is a must.

In summary, there remains a clear need for novel antibody-based compositions with specificity for SARS-CoV-2.

### SUMMARY OF THE INVENTION

It is an object of the present invention to provide a novel antibody-based binding composition with specificity for the SARS-CoV-2 spike receptor-binding domain. More specifically, it was an object of the present invention to provide novel multispecific antibody-based binding composition with specificity for at least two different epitopes on the SARS-CoV-2 spike receptor-binding domain.

The inventors have now surprisingly found that such novel antibody-based binding composition with specificity for the SARS-CoV-2 spike receptor-binding domain with properties superior to those of similar antibodies already approved for the treatment of patients with SARS-CoV-2 infections could be obtained by extensive search in the repertoire of memory B-cells of convalescent patients.

Accordingly, in a first aspect, the present invention relates to an antibody-based binding composition with specificity for the SARS-CoV-2 spike receptor-binding domain, wherein said antibody-based binding composition comprises a combination of variable domains selected from the group of
(i) a VH domain comprising at least residues 3 to 138 according to the AHo numbering system of the VH domain according to SEQ ID NO:1, and a VL domain comprising at least residues 3 to 138 according to the AHo numbering system of the VL domain according to SEQ ID NO:2;
(ii) a VH domain comprising at least residues 3 to 138 according to the AHo numbering system of the VH domain according to SEQ ID NO:3, and a VL domain comprising at least residues 3 to 138 according to the AHo numbering system of the VL domain according to SEQ ID NO:4; and
(iii) a VH domain comprising at least residues 3 to 138 according to the AHo numbering system of the VH domain according to SEQ ID NO:5, and a VL domain comprising at least residues 3 to 138 according to the AHo numbering system of the VL domain according to SEQ ID NO:6.

In a second aspect, the present invention relates to a multispecific antibody-based binding composition with specificity for at least two different epitopes on the SARS-CoV-2 spike receptor-binding domain, wherein said multispecific antibody-based binding composition comprises a first antibody-based binding composition, which is an antibody-based binding composition of the present invention, and at least a second antibody-based binding composition with specificity for an epitope on the SARS-CoV-2 spike receptor-binding domain, which is different from the epitope of said first antibody-based binding composition.

In a third aspect, the present invention relates to a nucleic acid encoding the antibody-based binding composition of the present invention, or the multispecific antibody-based binding composition of the present invention.

In a fourth aspect, the present invention relates to a pharmaceutical composition comprising the antibody-based binding composition of the present invention, the multispecific antibody-based binding composition of the present invention, or the nucleic acid of the present invention, and a pharmaceutically acceptable carrier.

In a fifth aspect, the present invention relates to the antibody-based binding composition of the present invention, the multispecific antibody-based binding composition of the present invention, the nucleic acid of the present invention, or the pharmaceutical composition of the present invention, for use in the prevention or treatment of an infection with SARS-CoV-2.

### BRIEF DESCRIPTION OF THE DRAWINGS

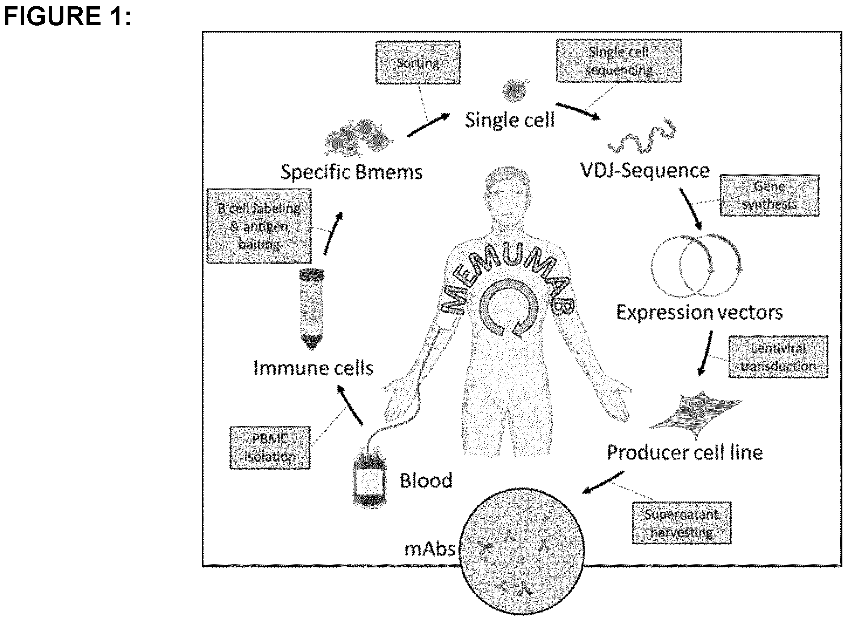

**FIGURE 1** shows a schematic view of the MEMUMAB-Pipeline. SARS-CoV-2-specific memory B cells from COVID-19 patients were labeled and sorted, and the underlying antibody code deciphered. Antibody sequences were generated by gene synthesis and respective expression cassettes delivered into producer cell lines. Screening of hundreds of candidates resulted in three very potent mAbs, which were highly effective and strongly neutralizing.

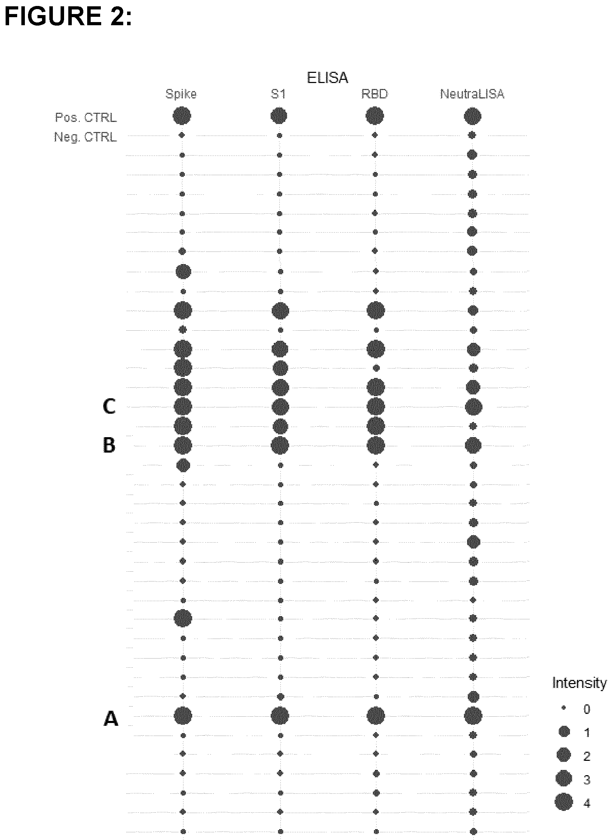

**FIGURE 2** shows an excerpt of the results obtained in the ELISA assay results described in Example 3.1. The left column shows the results with the full spike protein, followed by the results with the S1 domain, and the RBD polypeptide, respectively. The right column shows the results of the NeutraLISA experiments described in Example 3.2. The first two rows show the results with the positive control and the negative control, respectively. The following rows show the results for a variety of antibodies, including those of candidates A, B, and C, as indicated. The intensity of the ELISA signals is indicated by circles of different sizes.

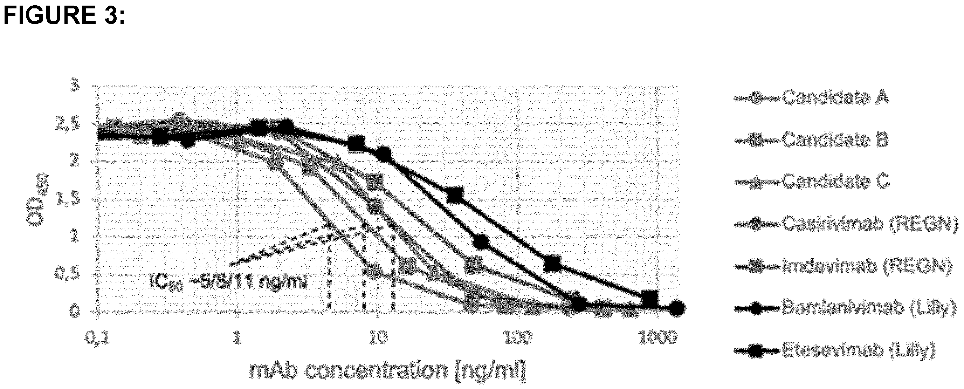

**FIGURE 3** shows a comparison of MEMUMAB candidates with already marketed SARS-CoV-2 neutralizing mAbs. A surrogate neutralization assay ("NeutraLISA") was used to quantify the neutralization capacity of each candidate. A decreasing signal indicates neutralization and occurs with increasing mAb concentrations. Concentration of half-maximal effect (IC₅₀) is lower in more potent mAbs.

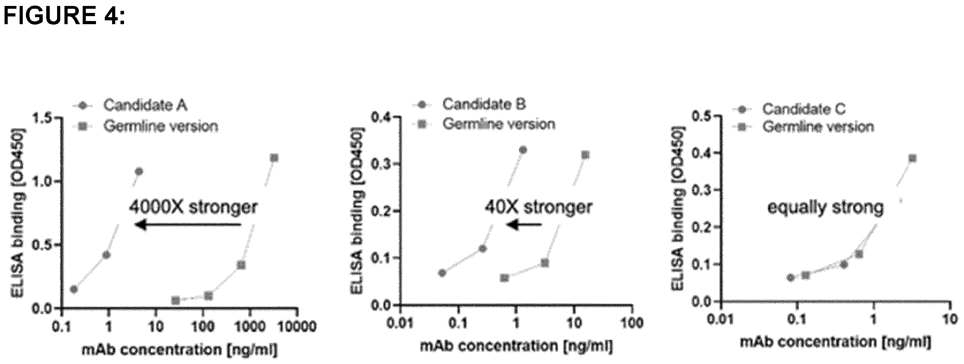

**FIGURE 4** shows the results of analyzing the effects of affinity maturation of SARS-CoV-2 neutralizing mAbs and that the strongest neutralizers also exhibit the strongest signs of somatic hypermutation. Each of the three candidates (A, B & C) was compared with its respective germline version in an S1 ELISA. Curves that are shifted to the left indicate improved binding.

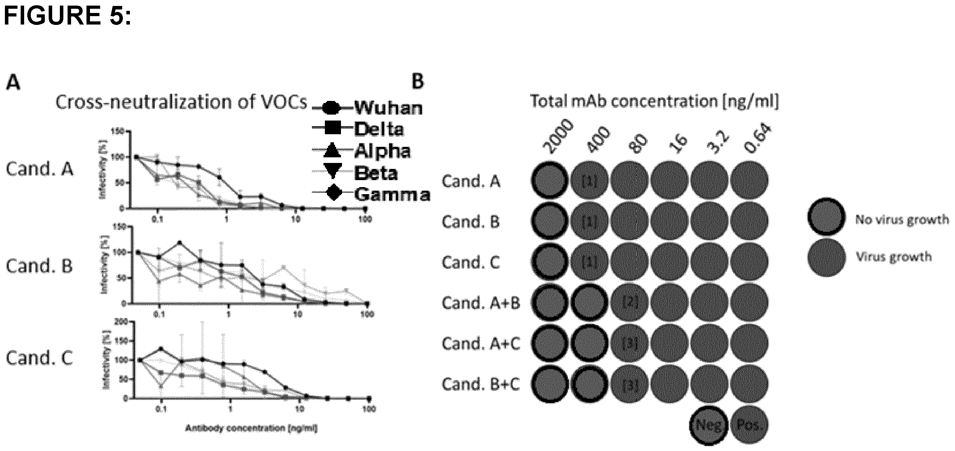

**FIGURE 5** shows that the mAb candidates cross-neutralize VOCs and cocktails of the three antibodies result in suppression of viral escape. (A) VSV pseudoparticles with different versions of the Spike protein were tested for susceptibility to neutralization by the mAb candidates. With increasing mAb concentrations (x-axis), all three mAb candidates cross-neutralize VOCs in a comparable potency as they neutralize the original virus. (B) Decreasing concentrations of the mAbs and their combinations were tested for VSV-Spike growth in cell culture. The virus which was isolated from the infected well with the highest mAb concentration did not show escape in follow-up experiments, indicating suppression of viral escape. In case of mAb combinations, the concentration represents the combined concentration of both mAbs, not of each mAb. [1] Mutations detected, escape verified, [2] mutation detected, no escape of the mutant virus observable in later experiments, [3] no mutation detected.

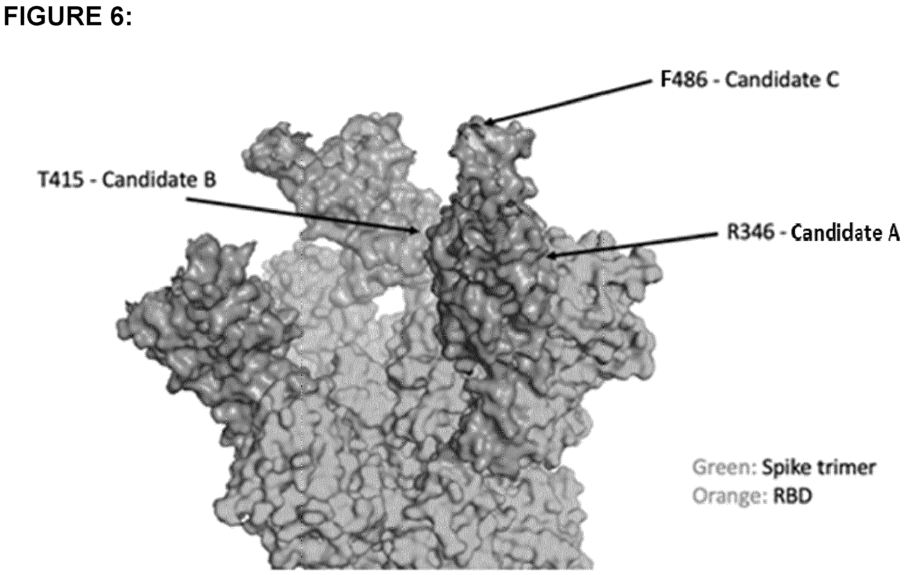

**FIGURE 6** shows the spatial location of amino acid residues that are important for neutralization. The identified positions of the escape mutations are highlighted in the SARS-CoV-2 spike structure.

### DETAILED DESCRIPTION OF THE INVENTION

While first antibody-based products with specificity for SARS-CoV-2 have already been approved in the US for the treatment of SARS-CoV-2 infected patients, based on promising results obtained in clinical trials, the trial results demonstrate that there is still substantial room for additional improvement. Furthermore, in light of the potential development of escape mutants that may render the approved drug cocktail ineffective, the development of back-up candidates is a must.

The present invention provides novel antibody-based binding composition with specificity for the SARS-CoV-2 spike receptor-binding domain that exhibit superior properties, in particular when used in combinations with each other. Those compositions of the present invention and pharmaceutical compositions comprising them thus provide distinct therapeutic advantages over compositions and therapies that have been established so far.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

Accordingly, in a first aspect, the present invention relates to an antibody-based binding composition with specificity for the SARS-CoV-2 spike receptor-binding domain, wherein said antibody-based binding composition comprises a combination of variable domains selected from the group of
(i) a VH domain comprising at least residues 3 to 138 according to the AHo numbering system of the VH domain according to SEQ ID NO:1, and a VL domain comprising at least residues 3 to 138 according to the AHo numbering system of the VL domain according to SEQ ID NO:2;
(ii) a VH domain comprising at least residues 3 to 138 according to the AHo numbering system of the VH domain according to SEQ ID NO:3, and a VL domain comprising at least residues 3 to 138 according to the AHo numbering system of the VL domain according to SEQ ID NO:4; and
(iii) a VH domain comprising at least residues 3 to 138 according to the AHo numbering system of the VH domain according to SEQ ID NO:5, and a VL domain comprising at least residues 3 to 138 according to the AHo numbering system of the VL domain according to SEQ ID NO:6.

The term "antibody", as used herein, refers naturally occurring glycoproteins that are central elements of the immune response to foreign substances. An antibody comprises at least two heavy (H) chains and two light (L) chains interconnected by disulfide bonds. Each heavy chain is comprised of a heavy chain variable domain (abbreviated herein as VH) and a heavy chain constant region. The heavy chain constant region is comprised of three domains, CH1, CH2 and CH3. Each light chain is comprised of a light chain variable domain (abbreviated herein as VL) and a light chain constant region. The light chain constant region is comprised of one domain (abbreviated herein as CL). The VH and VL regions can be further subdivided into regions of hypervariability, termed complementarity determining regions (CDRs), interspersed with regions that are more conserved, termed framework regions (FRs). Each VH and VL is composed of three CDRs and four FRs arranged from amino-terminus to carboxy-terminus in the following order: FR1, CDR1, FR2, CDR2, FR3, CDR3, FR4. The variable domains of the heavy and light chains form a binding domain that specifically interacts with an antigen. The constant regions of the antibodies may mediate the binding of the immunoglobulin to host tissues or factors, including various cells of the immune system (e. g., effector cells) and the first component (Clq) of the classical complement system.

The term "antibody-based binding composition" as used herein refers to any proteinaceous composition comprising at least a part of an antibody that is able to specifically bind to the antigen of that antibody, in the present case to the SARS-CoV-2 spike receptor-binding domain. Such part of an antibody may be referred to herein as "binding domain", "antigen-binding fragment", or "antigen-binding portion" of an antibody, and the like, as used herein. An antibody-based binding composition as used herein refers to compositions consisting of one antibody or compositions consisting of one or more fragments of an intact antibody that retain the ability to specifically bind to the antigen of that antibody, in the present case to the SARS-CoV-2 spike receptor-binding domain.

The term "binding specificity" as used herein refers to the ability of an individual antibody to react with one antigenic determinant and not with a different antigenic determinant. As used herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In its most general form (and when no defined reference is mentioned), "specific binding" refers to the ability of the antibody to discriminate between the target of interest and an unrelated molecule, as determined, for example, in accordance with specificity assay methods known in the art. Such methods comprise, but are not limited to Western blots, ELISA, RIA, ECL, IRMA, SPR (Surface plasmon resonance) tests and peptide scans. For example, a standard ELISA assay can be carried out. The scoring may be carried out by standard color development (*e*. *g*. secondary antibody with horseradish peroxide and tetramethyl benzidine with hydrogen peroxide). The reaction in certain wells is scored by the optical density, for example, at 450 nm. Typical background (= negative reaction) may be about 0.1 OD; the typical positive reaction may be about 1 OD. This means the ratio between a positive and a negative score can be 10-fold or higher. In a further example, an SPR assay can be carried out, wherein at least 10-fold, particularly at least 100-fold difference between a background and signal indicates on specific binding. Typically, determination of binding specificity is performed by using not a single reference molecule, but a set of about three to five unrelated molecules, such as milk powder, transferrin or the like.

The term "Complementarity Determining Regions" ("CDRs") refers to amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273, 927-948 ("Chothia" numbering scheme); ImMunoGenTics (IMGT) numbering (Lefranc, M.-P., The Immunologist, 7, 132-136 (1999); Lefranc, M.-P. et al., Dev. Comp. Immunol., 27, 55-77 (2003)) ("IMGT" numbering scheme); and the numbering scheme described in Honegger & Pluckthun, J. Mol. Biol. 309 (2001) 657-670 ("AHo" numbering). For example, for classic formats, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia, the CDR amino acids in the VH are numbered 26-32 (HCDR1), 52-56 (HCDR2), and 95-102 (HCDR3); and the amino acid residues in VL are numbered 24-34 (LCDR1), 50-56 (LCDR2), and 89-97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align.

In the context of the present invention, the numbering system suggested by Honegger & Pluckthun ("AHo") is used (Honegger & Pluckthun, J. Mol. Biol. 309 (2001) 657-670), unless specifically mentioned otherwise. In particular, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H32-H42; HCDR2 (also referred to as CDR-H2): H57-H76; HCDR3 (also referred to as CDR-H3): H109-H138. For the sake of clarity, the numbering system according to Honegger & Pluckthun takes the length diversity into account that is found in naturally occurring antibodies, both in the different VH and VL subfamilies and, in particular, in the CDRs, and provides for gaps in the sequences. Thus, in a given antibody variable domain usually not all positions 1 to 149 will be occupied by an amino acid residue.

As stated above, each of the variable domains VH and VL of antibodies comprises CDR regions CDR1 to CDR3, and framework regions FR1 to FR4. The CDRs are loop regions that together form the antigen-binding site, while the framework regions provide the globular structural backbone and additionally support the CDR loop regions, including certain residues that are critical for antigen-binding.

Based on the sequence homology of the framework regions, it is possible to group the variable domains into distinct variable domain families.

In the context of the present invention, the term "belonging to the VHx family" (or the VLx family) means that the framework sequences FR1 to FR3 show the highest degree of homology to said VHx family (or VLx, respectively). Examples of VH and VL families are given in Knappik et al., J. Mol. Biol. 296 (2000) 57-86, or in WO 2019/057787. In the context of the present invention, the set of VH and VL families as listed in Knappik et al. are used as reference sequences. The VH domains of each of the three candidates A to C of the present invention (SEQ ID NOs: 1, 5, and 9) belong to the VH3 family, whereas the VL domain of Candidate A (SEQ ID NO: 2) belongs to the Vkappa1 family, that of Candidate B (SEQ ID NO: 6) to the Vkappa3 family and that of Candidate C (SEQ ID NO: 10) to the Vlambda2 family.

While the grouping into families is determined based on the homology of, and while the residues in the framework regions that are critical for binding are comprised in, framework regions 1 to 3, the sequence of FR4 is less relevant, and FR4 sequences can be selected or exchanged more freely. This is not particularly surprising since antibody genes are generated in vivo by recombination of germline V, D, and J segments (VH domains) and V and J segments (VL domains), wherein the V segments encode frameworks 1 to 3, CDR1, CDR2 and part of CDR3, whereas FR4 is encoded by the J segments. Thus, while the variable domains of the antibody-based binding composition of the present invention are defined by reference to positions 3 to 138 only, *i*. *e*. a sequence stretch not including FR4, it is submitted that the antibody-based binding compositions of the present invention additionally comprise a FR4 region in each of VH and VL. In particular embodiments, that additional FR4 region is the FR4 region comprised in the corresponding sequence shown in Table 1, *i*. *e*. in the case of Candidate A (subpart (i) of [0031], the VH domain comprises at least residues 3 to 149 according to the AHo numbering system of the VH domain according to SEQ ID NO:1, and the VL domain comprising at least residues 3 to 149 according to the AHo numbering system of the VL domain according to SEQ ID NO:2, in the case of Candidate B (subpart (ii) of [0031], the VH domain comprises at least residues 3 to 149 according to the AHo numbering system of the VH domain according to SEQ ID NO:5, and the VL domain comprising at least residues 3 to 149 according to the AHo numbering system of the VL domain according to SEQ ID NO:6, and in the case of Candidate C (subpart (iii) of [0031], the VH domain comprises at least residues 3 to 149 according to the AHo numbering system of the VH domain according to SEQ ID NO:9, and the VL domain comprising at least residues 3 to 149 according to the AHo numbering system of the VL domain according to SEQ ID NO:10. Alternatively, the VH or VL domains of the present invention may comprise (a) an alternative FR4 region that is found in a rearranged antibody sequence belonging to the same VHx or VLx family, (b) an alternative FR4 region that is the sequence as present in one of the corresponding genomic J segments, or (c) a variant of the FR4 as present in Table 4, where such variant comprises one or two, particularly just one amino acid residue substitution. In that context it should be mentioned that it has even been shown that is possible to create hybrid variable domains comprising Vkappa-based frameworks FR1 to FR3 with a Vlambda-based FR4 sequences (see WO 2019/ 057787A1).

Similarly, amino acid residues at the N-terminus of the variable domains are less critical for the structural integrity of the variable domains and/or the binding properties. Thus, while the variable domains of the antibody-based binding composition of the present invention are defined by reference to positions 3 to 138 only, *i*. *e*. a sequence stretch not including the first two amino acid residues at the N-terminal ends, it is submitted that the antibody-based binding compositions of the present invention additionally comprise two amino acid residues at the N-terminal ends in each of VH and VL. In particular embodiments, those additional two amino acid residues at the N-terminal ends are the two amino acid residues at the N-terminal ends comprised in the corresponding sequence shown in Table 1, *i*. *e*. in the case of Candidate A (subpart (i) of [0031], the VH domain comprises at least residues 1 to 138 (or 149) according to the AHo numbering system of the VH domain according to SEQ ID NO:1, and the VL domain comprising at least residues 1 to 138 (or 149) according to the AHo numbering system of the VL domain according to SEQ ID NO:2, in the case of Candidate B (subpart (ii) of [0031], the VH domain comprises at least residues 1 to 138 (or 149) according to the AHo numbering system of the VH domain according to SEQ ID NO:5, and the VL domain comprising at least residues 1 to 138 (or 149) according to the AHo numbering system of the VL domain according to SEQ ID NO:6, and in the case of Candidate C (subpart (iii) of [0031], the VH domain comprises at least residues 1 to 138 (or 149) according to the AHo numbering system of the VH domain according to SEQ ID NO:9, and the VL domain comprising at least residues 1 to 138 (or 149) according to the AHo numbering system of the VL domain according to SEQ ID NO:10. Alternatively, the VH or VL domains of the present invention may comprise (a) an alternative N-terminal end that is found in a rearranged antibody sequence belonging to the same VHx or VLx family, (b) an alternative N-terminal end that is the sequence as present in one of the corresponding genomic J segments, or (c) a variant of the N-terminal end as present in Table 4, where such variant comprises one amino acid residue substitution. In that context, it should be mentioned that it has been shown by Knappik et al. (loc. cit.) that it is possible to generate and use a Vkappa3 consensus gene that starts with "DI..." as in the other Vkappa families instead of "EI..." as present in the repertoire of germline V segments belonging to the Vkappa3 family.

In a particular aspect, the present invention relates to a variant of an antibody-based binding composition as defined hereinabove, wherein such variant is still specific for the SARS-CoV-2 spike receptor-binding domain, but comprises at most eight amino acid residue substitutions compared to the corresponding sequence of SEQ ID NOs: 1, 2, 5, 6, 9 or 10, particularly at most six amino acid residue substitutions, more particularly at most five, four, three, or two amino acid residue substitutions. In particular embodiments, the variant comprises one amino acid residue substitution. In particular embodiments, the substitutions only relate to non-canonical residues in the framework regions. In the context of the present invention, the term "canonical residue" refers to positions that are involved in stabilizing or supporting the particular three-dimensional presentation and arrangement of the CDR loops. The nature and position of the canonical residues are well known to one of ordinary skill in the art, and may, for example, be found in Knappik et al. (loc. cit.).

In particular embodiments, a substitution is the substitution of an amino acid residue present in a sequence according to SEQ ID NOs: 1, 2, 5, 6, 9 or 10, by an alternative residue that is either more often found at that position for the corresponding VHx or VLx family in the list of rearranged Vkappa, Vlambda and VH sequences present in Tables 4 to 6 of WO 97/08320, or, when the amino acid residue present in a sequence according to SEQ ID NOs: 1, 2, 5, 6, 9 or 10 is the most frequently found amino acid residue at that position, by the amino acid residue that is found with the second-highest frequency.

In a particular embodiment, said antibody-based binding composition is a full-length antibody, particularly a full-length antibody selected from the list of IgA, IgD, IgE and IgG.

In a particular embodiment, said full-length antibody is an IgG, particularly an IgG selected from the list of IgG1, IgG2, IgG3 and IgG4.

In order to increase the number of specificities/functionalities at the same or lower molecular weight, it is advantageous to use antibody-based compositions comprising antibody fragments, such as Fv, scFv, Fab, Fab' and F(ab')₂ fragments and other antibody fragments. These smaller molecules retain the antigen-binding activity of the whole antibody and can also exhibit improved tissue penetration and pharmacokinetic properties in comparison to the whole immunoglobulin molecules and thus hold the promise for improved efficacy at the same or lower dose.

Thus, in a particular other embodiment, said antibody-based binding composition is an antibody-based antigen-binding fragment, particularly an antigen-binding fragment selected from the list of: Fab fragment, F(ab)2 fragment, Fv fragment, dsFv fragment, scFv fragment, a dsscFv fragment, and a fusion protein comprising a Fab fragment, a F(ab)2 fragment, an Fv fragment, a dsFv fragment, an scFv fragment or an dsscFv, particularly wherein said antigen-binding fragment is an scFv fragment or a fusion protein comprising an scFv fragment, particularly an scFv-Fc fusion protein or an (scFv)₂-Fc fusion protein.

In the context of the present invention, the term "Fab fragment" refers to a monovalent fragment consisting of the VL, VH, CL and CH1 domains; a "Fab' fragment" refers to a Fab fragment additionally comprising part of an antibody hinge region, a "F(ab')₂ fragment" is a bivalent fragment comprising two Fab fragments linked by a disulfide bridge at the hinge region; an "Fv fragment" consists of the VL and VH domains of a single arm of an antibody; a "dsFv" refers to an Fv fragment linked by a disulfide bond, tem "scFv fragment" refers to a single-chain Fv fragment, wherein the VL and VH domains are connected by a polypeptide linker, and the term "dsscFv fragment" refers to an scFv fragment, wherein the two variable domains are additionally linked by a disulfide bridge.

In the context of the present invention, the term "polypeptide linker" refers to a linker consisting of a chain of amino acid residues linked by peptide bonds that is connecting two domains, each being attached to one end of the linker. The polypeptide linker should have a length that is adequate to link two molecules in such a way that they assume the correct conformation relative to one another so that they retain the desired activity. In particular embodiments, the polypeptide linker has a continuous chain of between 2 and 30 amino acid residues (*e*. *g.*, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acid residues). In addition, the amino acid residues selected for inclusion in the polypeptide linker should exhibit properties that do not interfere significantly with the activity of the polypeptide. Thus, the linker peptide on the whole should not exhibit a charge that would be inconsistent with the activity of the polypeptide, or interfere with internal folding, or form bonds or other interactions with amino acid residues in one or more of the monomers that would seriously impede the binding of receptor monomer domains. In particular embodiments, the polypeptide linker is non-structured polypeptide. Useful linkers include glycine-serine, or GS linkers. By "Gly-Ser" or "GS" linkers is meant a polymer of glycines and serines in series (including, for example, (Gly-Ser)ₙ, (GSGGS)ₙ (GGGGS)ₙ and (GGGS)ₙ, where n is an integer of at least one), glycine-alanine polymers, alanine-serine polymers, and other flexible linkers such as the tether for the shaker potassium channel, and a large variety of other flexible linkers, as will be appreciated by those in the art. Glycine-serine polymers are preferred since oligopeptides comprising these amino acids are relatively unstructured, and therefore may be able to serve as a neutral tether between components. Secondly, serine is hydrophilic and therefore able to solubilize what could be a globular glycine chain. Third, similar chains have been shown to be effective in joining subunits of recombinant proteins such as single-chain antibodies.

In a preferred embodiment, the binding domain of an antibody applied in the present invention is a single-chain Fv fragment (scFv).

In particular embodiments, the two variable domains of an antigen-binding fragment, as in an Fv or an scFv fragment, are stabilized by an interdomain disulfide bond, in particular, said VH domain comprises a single cysteine residue in position 51 (AHo numbering) and said VL domain comprises a single cysteine residue in position 141 (AHo numbering).

The term "immunoglobulin Fc region", or "Fc region" or "Fc" in abbreviated form, as used herein, refers to the CH2 and CH3 domains of the heavy chain constant regions.

In particular embodiments, the antibody-based binding composition of the present invention or the multispecific antibody-based binding composition of the present invention comprises an immunoglobulin Fc region polypeptide. The term "Fc region" herein is used to define a C-terminal region of an immunoglobulin heavy chain, including native-sequence Fc regions and variant Fc regions. Native-sequence Fc regions include human IgG1, IgG2 (IgG2A, IgG2B), IgG3 and IgG4. "Fc receptor" or "FcR" describes a receptor that binds to the Fc region of an antibody. Particularly, the FcR is a native sequence human FcR, which binds an IgG antibody (a gamma receptor) and includes receptors of the FcγRI, FcyRII, and FcγRIII subclasses, including allelic variants and alternatively spliced forms of these receptors, FcγRII receptors including FcyRIIA (an "activating receptor") and FcγRI IB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see M. Daeron, Annu. Rev. Immunol. 5:203-234 (1997). FcRs are reviewed in Ravetch and Kinet, Annu. Rev. Immunol. 9: 457-92 (1991); Capet et al, Immunomethods 4: 25-34 (1994); and de Haas et al, J. Lab. Clin. Med. 126: 330-41 (1995). Other FcRs, including those to be identified in the future, are encompassed by the term "FcR" herein. The term "Fc receptor" or "FcR" also includes the neonatal receptor, FcRn, which is responsible for the transfer of maternal IgGs to the fetus. Guyer et al., J. Immunol. 117: 587 (1976) and Kim et al., J. Immunol. 24: 249 (1994). Methods of measuring binding to FcRn are known (see, *e*. *g*., Ghetie and Ward, Immunol. Today 18: (12): 592-8 (1997); Ghetie et al., Nature Biotechnology 15 (7): 637-40 (1997); Hinton et al., J. Biol. Chem. TJI (8): 6213-6 (2004); WO 2004/92219 (Hinton et al). Binding to FcRn *in vivo* and serum half-life of human FcRn high-affinity binding polypeptides can be assayed, *e*. *g*., in transgenic mice or transfected human cell lines expressing human FcRn, or in primates to which the polypeptides having a variant Fc region are administered. WO 2004/42072 (Presta) describes antibody variants that improved or diminished binding to FcRs. See also, *e*. *g*., Shields et al., J. Biol. Chem. 9(2): 6591-6604 (2001).

In a particular embodiment, said antibody-based antigen-binding fragment is an scFv fragment selected from the list of scFv fragments according to SEQ ID NOs: 13 to 18.

In particular embodiments of the particular aspect presented in [0042], where said antibody-based binding composition is an antibody-based antigen-binding fragment, the present invention relates to a variant of an antibody-based binding composition as defined hereinabove, wherein such variant is still specific for the SARS-CoV-2 spike receptor-binding domain, but comprises at most eight amino acid residue substitutions compared to the corresponding sequence of SEQ ID NOs: 1, 2, 5, 6, 9 or 10, particularly at most six amino acid residue substitutions, more particularly at most five, four, three, two or just one amino acid residue substitution(s), wherein such substitutions are performed at positions in a variable domain, which are no longer in their native context, when present in such antigen-binding fragment. Examples include variable domain residues originally present in the interface between variable and constant domain, which become solvent-exposed when present in an Fv or scFv fragment. The identification of such residues and approaches for identifying suitable substitutions are well known to one of ordinary skill in the art (see, for example, Ewert et al., Biochemistry 42 (2003) 1517-1528; Worn & Plückthun, J. Mol. Biol. 305 (2001) 989-1010; Nieba et al., Protein Eng. 10 (1997) 435-444). Additionally, substitutions may be performed at positions that are known or suspected to result in improved biochemical properties (see, for, example, WO 2009/000099) or result in decreased immunogenicity (see, for example, WO 2011/075861).

Suitably, the antibody-based binding composition of the present invention is an isolated antibody-based binding composition. The term "isolated antibody-based binding composition", as used herein, refers to a composition that is substantially free of other antibody-based products having different antigenic specificities (*e*. *g*., an isolated antibody-based binding composition that specifically binds SARS-CoV-2 spike receptor-binding domain is substantially free of antibodies that specifically bind antigens other than SARS-CoV-2 spike receptor-binding domain, an isolated antibody-based binding composition that specifically binds to two or three different epitopes on SARS-CoV-2 spike receptor-binding domain is substantially free of antibodies that specifically bind antigens other than any of these two or three epitopes on SARS-CoV-2 spike receptor-binding domain). Moreover, an isolated antibody-based binding composition may be substantially free of other cellular material and/or chemicals.

In a second aspect, the present invention relates to a multispecific antibody-based binding composition with specificity for at least two different epitopes on the SARS-CoV-2 spike receptor-binding domain, wherein said multispecific antibody-based binding composition comprises a first antibody-based binding composition, which is an antibody-based binding composition of the present invention, and at least a second antibody-based binding composition with specificity for an epitope on the SARS-CoV-2 spike receptor-binding domain, which is different from the epitope of said first antibody-based binding composition.

The term "multispecific antibody-based binding composition" as used herein, refers to an antibody-based binding composition that binds to two or more different epitopes on the SARS-CoV-2 spike receptor-binding domain. The term "multispecific antibody-based binding composition" includes bispecific, trispecific, tetraspecific, pentaspecific and hexaspecific. The term "bispecific antibody" as used herein, refers to an antibody that binds to at least two different epitopes on on the SARS-CoV-2 spike receptor-binding domain. The term "trispecific antibody" as used herein, refers to an antibody that binds to at least three different epitopes on the SARS-CoV-2 spike receptor-binding domain.

The term "epitope" means a protein determinant capable of specific binding to an antibody. Epitopes usually consist of chemically active surface groupings of molecules such as amino acids or sugar side chains and usually have specific three-dimensional structural characteristics, as well as specific charge characteristics. "Conformational" and "linear" epitopes are distinguished in that the binding to the former but not the latter is lost in the presence of denaturing solvents.

The term "conformational epitope" as used herein refers to amino acid residues of an antigen that come together on the surface when the polypeptide chain folds to form the native protein.

The term "linear epitope" refers to an epitope, wherein all points of interaction between the protein and the interacting molecule (such as an antibody) occurring linearly along the primary amino acid sequence of the protein (continuous).

The term "recognize" as used herein refers to an antibody antigen-binding fragment thereof that finds and interacts (*e*. *g*., binds) with its conformational epitope.

The term "avidity" as used herein refers to an informative measure of the overall stability or strength of the antibody-antigen complex. It is controlled by three major factors: antibody epitope affinity, the valency of both the antigen and antibody, and the structural arrangement of the interacting parts. Ultimately these factors define the specificity of the antibody, that is, the likelihood that the particular antibody is binding to a precise antigen epitope.

The term "same epitope", as used herein, refers to individual protein determinants on the same protein capable of specific binding to an antibody, where these individual protein determinants are identical, *i*. *e*. consist of identical chemically active surface groupings of molecules such as amino acids or sugar side chains having identical three-dimensional structural characteristics, as well as identical charge characteristics. The term "different epitope", as used herein in connection with a specific protein target, refers to individual protein determinants on the same protein capable of specific binding to an antibody, where these individual protein determinants are not identical, *i*. *e*. consist of non-identical chemically active surface groupings of molecules such as amino acids or sugar side chains having different three-dimensional structural characteristics, as well as different charge characteristics. These different epitopes can be overlapping or non-overlapping.

As used herein, the term "affinity" refers to the strength of interaction between antibody and antigen at single antigenic sites. Within each antigenic site, the variable region of the antibody "arm" interacts through weak non-covalent forces with antigen at numerous sites; the more interactions, the stronger the affinity.

"Binding affinity" generally refers to the strength of the total sum of non-covalent interactions between a single binding site of a molecule (*e*. *g*., of an antibody) and its binding partner (*e*. *g*., an antigen or, more specifically, an epitope on an antigen). Unless indicated otherwise, as used herein, "binding affinity", "bind to", "binds to" or "binding to" refers to intrinsic binding affinity that reflects a 1:1 interaction between members of a binding pair (*e*. *g*., an antibody fragment and antigen). The affinity of a molecule X for its partner Y can generally be represented by the dissociation constant (K_{D}). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative and exemplary embodiments for measuring binding affinity, *i*. *e*. binding strength are described in the following.

The term "K_{assoc}", "Kₐ" or "Kₒₙ", as used herein, are intended to refer to the association rate of a particular antibody-antigen interaction, whereas the term "K_{dis}", "K_{d}" or "K_{off}", as used herein, is intended to refer to the dissociation rate of a particular antibody- antigen interaction. In one embodiment, the term "K_{D}", as used herein, is intended to refer to the dissociation constant, which is obtained from the ratio of K_{d} to Kₐ (*i*. *e*. K_{d}/Kₐ) and is expressed as a molar concentration (M). The "K_{D}" or "K_{D} value" or "KD" or "KD value" according to this invention is in one embodiment measured by using surface-plasmon resonance assays.

In a particular embodiment, said second antibody-based binding composition is an antibody-based binding composition of the present invention.

In a particular such embodiment, said multispecific antibody-based binding composition comprises a combination of antibody Candidate A with antibody Candidate B, a combination of antibody Candidate A with antibody Candidate C, a combination of antibody Candidate B with antibody Candidate C, or a combination of antibody Candidate A with antibody Candidates B and C.

In a particular embodiment, said second antibody-based binding composition is an antibody-based binding composition from a different source, in particular a binding composition based on an antibody selected from Casirivimab and Imdevimab.

In a particular embodiment, said first and said second antibody-based binding compositions are present as two separate monospecific constructs.

In a particular other embodiment, said first and said second antibody-based binding compositions are comprised in one multispecific construct, particularly a multispecific construct selected from the list of: diabody; scDb; tandem di-scFv, tandem tri-scFv; Fab-scFv; scFab-dsscFv; Fab-(scFv)₂; (Fab')₂; Fab-(Fv)₂; triabody; scDb-scFv; tandem tri-scFv, a scFab-dsscFv, and constructs comprising two antigen-based binding compositions selected from Fab fragment, F(ab')₂ fragment, Fv fragment, dsFv fragment, and scFv fragment, wherein each of said two antigen-based binding compositions is fused to the N- and/or the C-terminus of one chain of a heterodimerization domain, particularly one chain of a heterodimeric Fc domain.

The term "diabody" refers to an antibody fragment with two antigen-binding sites, which fragments comprise a VH connected to VL in the same polypeptide chain (VH-VL). By using a linker that is too short to allow pairing between the two domains on the same chain, the domains are forced to pair with the complementary domains of another chain to create two antigen-binding sites. A diabody may be bivalent or bispecific. Diabodies are described more fully in, for example, EP 404 097, WO 93/01161, Hudson et al., Nat. Med. 9:129-134 (2003), and Holliger et al., Proc. Natl. Acad. Sci. USA 90: 6444-6448 (1993). Triabodies and tetrabodies are also described in Hudson et al., Nat. Med. 9:129-134 (2003).

The term "scDb" refers to a single-chain diabody, which comprises two variable heavy chain domains (VH) or fragments thereof and two variable light chain domains (VL) or fragments thereof connected by linkers L1, L2 and L3 in the order VHA-L1-VLB-L2-VHB-L3-VLA, VHA-L1-VHB-L2-VLB-L3-VLA, VLA-L1-VLB-L2-VHB-L3-VHA, VLA-L1-VHB-L2-VLB-L3-VHA, VHB-L1-VLA-L2-VHA-L3-VLB, VHB-L1-VHA-L2-VLA-L3-VLB, VLB-L1-VLA-L2-VHA-L3-VHB or VLB-L1-VHA-L2-VLA-L3-VHB, wherein the VLA and VHA domains jointly form the antigen-binding site for the first antigen, and VLB and VHB jointly form the antigen-binding site for the second antigen.

The linker L1 particularly is a peptide of 2-10 amino acids, more particularly 3-7 amino acids, and most particularly 5 amino acids, and linker L3 particularly is a peptide of 1-10 amino acids, more particularly 2-7 amino acids, and most particularly 5 amino acids. In particular embodiments, the linker L1 and/or L3 comprises one or two units of four (4) glycine amino acid residues and one (1) serine amino acid residue (GGGGS)ₙ, wherein n=1 or 2, particularly n=1.

The middle linker L2 particularly is a peptide of 10-40 amino acids, more particularly 15-30 amino acids, and most particularly 20-25 amino acids. In particular embodiments, said linker L2 comprises two or more units of four (4) glycine amino acid residues and one (1) serine amino acid residue (GGGGS)ₙ, wherein n= 2, 3, 4, 5, 6, 7 or 8, particularly n=4 or 5.

The term "scDb-scFv" refers to an antibody format, wherein a single-chain Fv (scFv) fragment is fused by a flexible Gly-Ser linker to a single-chain diabody (scDb). In one embodiment, said flexible Gly-Ser linker is a peptide of 2-40 amino acids, *e*. *g*., 2-35, 2-30, 2-25, 2-20, 2-15, 2-10 amino acids, particularly 10 amino acids. In particular embodiments, said linker comprises one or more units of four (4) glycine amino acid residues and one (1) serine amino acid residue (GGGGS)ₙ, wherein n=1, 2, 3, 4, 5, 6, 7 or 8, particularly n=2.

In particular other embodiments, the multispecific antibody-based binding composition is in a format selected from a multispecific, *e*. *g*. at least bispecific, format selected from the list of a tandem scDb (Tandab); tetrabody; scDb-scFv; di-diabody; scFv-Fc-scFv fusion (ADAPTIR); DVD-Ig; IgG-scFv fusions, such as CODV-IgG, Morrison (IgG CH₃-scFv fusion (Morrison L) or IgG CL-scFv fusion (Morrison H)), bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain) and Ts2Ab (dsscFv linked to C-terminus of heavy chain); a DART^{™}; a TRIDENT^{™}; a scDb, a tandem tri-scFv, a MATCH (described in WO 2016/0202457; Egan T. et al., MABS 9 (2017) 68-84) and DuoBodies (bispecific IgGs prepared by the Duobody technology) (MAbs. 2017 Feb/Mar;9(2):182-212. doi: 10.1080/19420862.2016.1268307).

Suitably, the binding domains of the multispecific antibody-based binding composition are operably linked.

The term "operably linked", as used herein, indicates that two molecules (*e*. *g*., polypeptides, domains, binding domains) are attached in a way that each molecule retains functional activity. Two molecules can be "operably linked" whether they are attached directly or indirectly (*e*. *g*., via a linker, via a moiety, via a linker to a moiety). The term "linker" refers to a peptide or other moiety that is optionally located between binding domains or antibody fragments used in the invention. A number of strategies may be used to covalently link molecules together. These include, but are not limited to, polypeptide linkages between N- and C-termini of proteins or protein domains, linkage via disulfide bonds, and linkage via chemical cross-linking reagents. In one aspect of this embodiment, the linker is a peptide bond generated by recombinant techniques or peptide synthesis. Choosing a suitable linker for a specific case where two polypeptide chains are to be connected depends on various parameters, including but not limited to the nature of the two polypeptide chains (*e*. *g*., whether they naturally oligomerize), the distance between the N- and the C-termini to be connected if known, and/or the stability of the linker towards proteolysis and oxidation. Furthermore, the linker may contain amino acid residues that provide flexibility.

The binding domains of the multispecific antibody-based binding composition of the present invention may be able to bind to SARS-CoV-2 spike receptor-binding domain in an alternative binding mode or independent of each other and thus simultaneously. The term" alternative binding mode" refers to a situation, where binding of one epitope on SARS-CoV-2 spike receptor-binding domain by the corresponding binding domain of the multispecific antibody-based binding composition of the present invention blocks binding of a second binding domain of the of said multispecific antibody-based binding composition to its epitope. In contrast, the term "simultaneously", as used in this connection refers to the simultaneous binding of at least two binding domains to their respective epitopes on the SARS-CoV-2 spike receptor-binding domain. In the case of alternative binding, one multispecific antibody-based binding composition of the present invention is expected to be able to neutralize two virus particles. In the case of simultaneous binding, an increased binding strength to one virus particle is to be expected due to avidity effects.

In particular embodiments, the antibody-based binding composition my further comprise an additional functional domain that is not related to binding to SARS-CoV-2 spike receptor-binding domain. In particular embodiment, such additional functional domain is increasing the half-life of the antibody-based binding composition of the present invention. In particular such embodiments, the additional functional domain is a human serum albumin binding domain (hSA-BD) having a specificity to human serum albumin.

The term "hSA" refers in particular to human serum albumin with UniProt ID number P02768. Human Serum Albumin (hSA) is a 66.4 kDa abundant protein in human serum (50 % of total protein) composed of 585 amino acids (Sugio, Protein Eng, Vol. 12, 1999, 439-446). Multifunctional hSA can transport a number of metabolites such as fatty acids, metal ions, bilirubin and some drugs (Fanali, Molecular Aspects of Medicine, Vol. 33, 2012, 209-290). hSA concentration in serum is around 3.5 - 5 g/dl. Albumin-binding antibodies and fragments thereof may be used for example, for extending the *in vivo* serum half-life of drugs or proteins conjugated thereto.

Suitable hSA-BDs comprises or is derived from a binding domain selected from the group consisting of: (i) polypeptides that bind serum albumin (see, for example, Smith et al., 2001, Bioconjugate Chem. 12:750-756; EP 0 486 525; US 6,267,964; WO 2004/001064; WO 2002/076489; and WO 2001/45746); (ii) antiserum albumin binding single variable domains described in Holt et al., Protein Engineering, Design & Selection, vol 21, 5, pp283-288, WO 2004/003019, WO 2008/096158, WO 2005/118642, WO 2006/0591056 and WO 2011/006915; (iii) antiserum albumin antibodies described in WO 2009/040562, WO 2010/035012 and WO 2011/086091.

In a third aspect, the present invention relates to a nucleic acid encoding the antibody-based binding composition of the present invention, or the multispecific antibody-based binding composition of the present invention.

The antibody-based binding composition of the present invention, or the multispecific antibody-based binding composition of the present invention can be produced using any convenient antibody-manufacturing method known in the art (see, *e*. *g*., Fischer, N. & Leger, O., Pathobiology 74 (2007) 3-14 with regard to the production of bispecific constructs; Hornig, N. & Farber-Schwarz, A., Methods Mol. Biol. 907 (2012)713-727, and WO 99/57150 with regard to bispecific diabodies and tandem scFvs). Specific examples of suitable methods for the preparation of the bispecific construct further include, inter alia, the Genmab (see Labrijn et al., Proc. Natl. Acad. Sci. USA 110 (2013) 5145-5150) and Merus (see de Kruif et al., Biotechnol. Bioeng. 106 (2010) 741-750) technologies. Methods for production of bispecific antibodies comprising a functional antibody Fc part are also known in the art (see, *e*. *g*., Zhu et al., Cancer Lett. 86 (1994) 127-134); and Suresh et al., Methods Enzymol. 121 (1986) 210-228).

In the case of multispecific antibody-based binding composition of the present invention, the combination of the antigen-binding domains or fragments or parts thereof of two or more different monoclonal antibodies can be prepared by conjugating the constituent binding specificities, using methods known in the art. For example, they can be conjugated by sulfhydryl bonding of the C-terminus hinge regions of the two heavy chains. In a particular embodiment, the hinge region is modified to contain an odd number of sulfhydryl residues, for example one, prior to conjugation.

Alternatively, two or more binding specificities can be encoded in the same vector and expressed and assembled in the same host cell. This method is particularly useful where the bispecific molecule is a mAb × mAb, mAb × Fab, Fab × F (ab')₂ or ligand × Fab fusion protein. The multispecific antibody used in the invention can be a single chain multispecific antibody comprising at least two binding determinants. The multispecific antibody use in the invention can also comprise at least two of said single-chain molecules. Methods for preparing multispecific antibodies and molecules are described, for example, in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the multispecific antibodies to their specific targets can be confirmed by, for example, enzyme-linked immunosorbent assay (ELISA), radioimmunoassay (REA), FACS analysis, bioassay (*e*. *g*., growth inhibition), or Western Blot assay. Each of these assays generally detects the presence of protein-antibody complexes of particular interest by employing a labeled reagent (e. g., an antibody) specific for the complex of interest.

In particular embodiments, the nucleic acid comprises a contiguous nucleic acid encoding the complete antibody-based binding composition of the present invention. In particular other embodiments, the nucleic acid consists of a single nucleic acid comprising two or more separate nucleic acid regions encoding two or more components of the complete antibody-based binding composition of the present invention. In particular other embodiments, the nucleic acid consists of two or more single nucleic acids, each encoding one of two or more components of the complete antibody-based binding composition of the present invention.

In particular embodiments, the nucleic acid is selected from the group of mRNA, nucleic acid comprised in a retroviral, e.g. gammaretroviral, alpharetroviral, or lentiviral vector system, or in an AAV-based vector system, and nucleic acid comprised in a vector comprised in a virus-like particle.

In particular such embodiments, said mRNA is suitable for the *in vivo* expression of the antibody-based binding composition of the present invention, or of the multispecific antibody-based binding composition of the present invention.

In particular such embodiments, said lentiviral or AAV-based vector system is suitable for the *in vivo* expression of the antibody-based binding composition of the present invention, or of the multispecific antibody-based binding composition of the present invention.

In a fourth aspect, the present invention relates to a pharmaceutical composition comprising the antibody-based binding composition of the present invention, the multispecific antibody-based binding composition of the present invention, or the nucleic acid of the present invention, and a pharmaceutically acceptable carrier.

"Pharmaceutically acceptable carrier" means a medium or diluent that does not interfere with the structure of the antibodies. Pharmaceutically acceptable carriers enhance or stabilize the composition, or facilitate preparation of the composition. Certain of such carriers enable pharmaceutical compositions to be formulated as, for example, tablets, pills, dragees, capsules, liquids, gels, syrups, slurries, suspension and lozenges for the oral ingestion by a subject. Certain of such carriers enable pharmaceutical compositions to be formulated for injection, infusion or topical administration. For example, a pharmaceutically acceptable carrier can be a sterile aqueous solution.

Pharmaceutically acceptable carriers include but are not limited to solvents, buffer solutions, dispersion media, coatings, antibacterial and antifungal agents, isotonic and absorption delaying agents, and the like that are physiologically compatible. In the context of pharmaceutical compositions based on mRNA, said pharmaceutically acceptable carriers include but are not limited to lipids and polymeric compounds forming liposomes and polymersomes, respectively.

The pharmaceutical compositions of the present invention can be prepared in accordance with methods well known and routinely practiced in the art. See, *e*. *g.*, Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the antibody-based binding composition of the present invention, or the multispecific antibody-based binding composition of the present invention is employed in the pharmaceutical compositions of the invention. The multispecific antibodies are formulated into pharmaceutically acceptable dosage forms by conventional methods known to those of skill in the art. Dosage regimens are adjusted to provide the optimum desired response (*e*. *g.*, a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions or the kit can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions used in the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

The pharmaceutical compositions or the kit of the invention can be administered by a variety of methods known in the art. In the case of administering the multispecific antibody components of the kits, administration may be done concomitantly or sequentially. In the case of a sequential administration, the multispecific antibody components may be administered based on individually adjusted administration schemes and regimens. The route and/or mode of administration vary depending upon the desired results. Administration can be intravenous, intramuscular, intraperitoneal, or subcutaneous, or administered proximal to the site of the target. In the context of the present invention, administration by the intranasal or inhalative route is particularly preferred. The pharmaceutically acceptable carrier should be suitable for intravenous, intramuscular, subcutaneous, parenteral, spinal or epidermal administration (*e*. *g.*, by injection or infusion). Depending on the route of administration, the active compound, *i*. *e*., the antibody-based binding composition of the present invention, the multispecific antibody-based binding composition of the present invention applied in the pharmaceutical composition of the invention, may be coated in a material to protect the compound from the action of acids and other natural conditions that may inactivate the compound.

The pharmaceutical composition of the invention is usually administered on multiple occasions. Intervals between single dosages can be daily, weekly, monthly or yearly. Intervals can also be irregular as indicated by measuring blood levels of the antibody-based binding composition of the present invention or the multispecific antibody-based binding composition of the present invention in the patient. Alternatively, the pharmaceutical composition of the invention can be administered as a sustained release formulation, in which case less frequent administration is required. Dosage and frequency vary depending on the half-life of the antibodies in the patient. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

In a particular embodiment, said pharmaceutically acceptable carrier is a carrier that is suitable for intranasal or inhalative application of said pharmaceutical composition.

In a fifth aspect, the present invention relates to the antibody-based binding composition of the present invention, the multispecific antibody-based binding composition of the present invention, the nucleic acid of the present invention, or the pharmaceutical composition of the present invention, for use in the prevention or treatment of an infection with SARS-CoV-2.

In another aspect, the present invention relates to a method of prophylactically treating a subject having a risk of infection with SARS-CoV-2, comprising administering to the subject a therapeutically effective amount of the pharmaceutical composition of the present invention.

In another aspect, the present invention relates to a method of treating a subject having been infected with SARS-CoV-2, comprising administering to the subject a therapeutically effective amount of the pharmaceutical composition of the present invention.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, *e*. *g.*, in a human, and includes: (a) inhibiting the disease, *i*. *e*., arresting its development; and (b) relieving the disease, *i*. *e*., causing regression of the disease.

The term "therapeutically effective amount" or "efficacious amount" refers to the amount of an agent that, when administered to a subject for treating a disease, is sufficient to affect such treatment for the disease. The "therapeutically effective amount" will vary depending on the agent, the disease and its severity and the age, weight, etc., of the subject to be treated.

### Sequence listing (mutations designated according to AHo numbering scheme; the CDRs defined according to Numab CDR definition, unless specified otherwise)

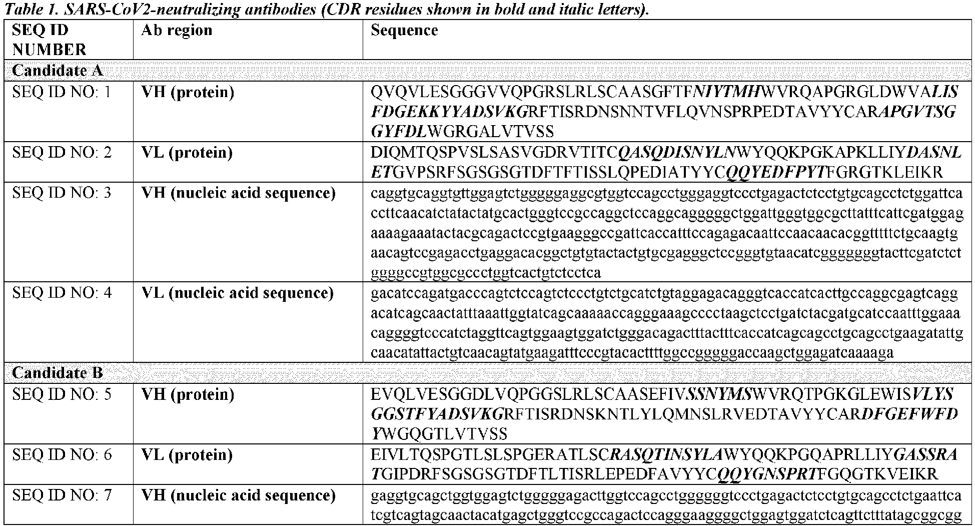

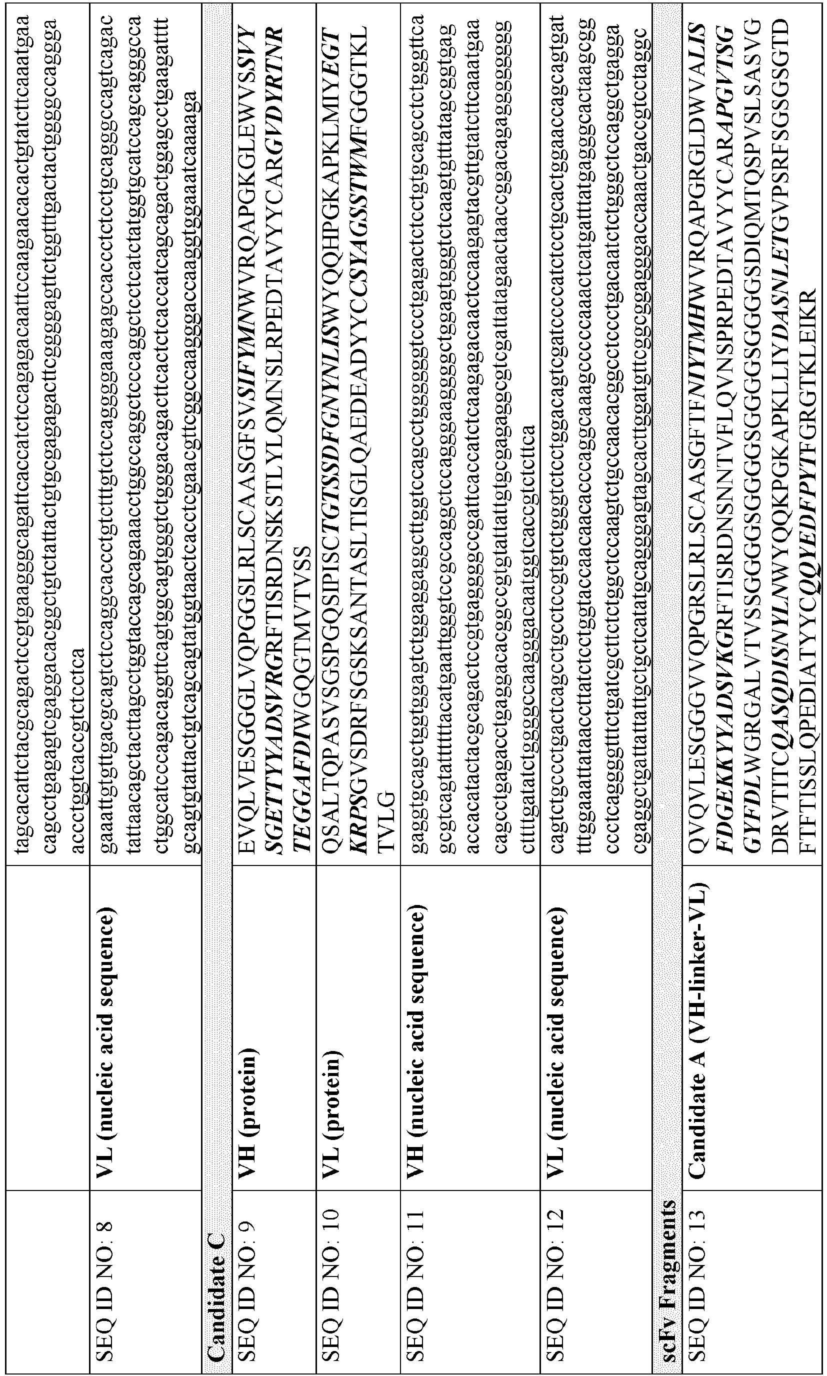

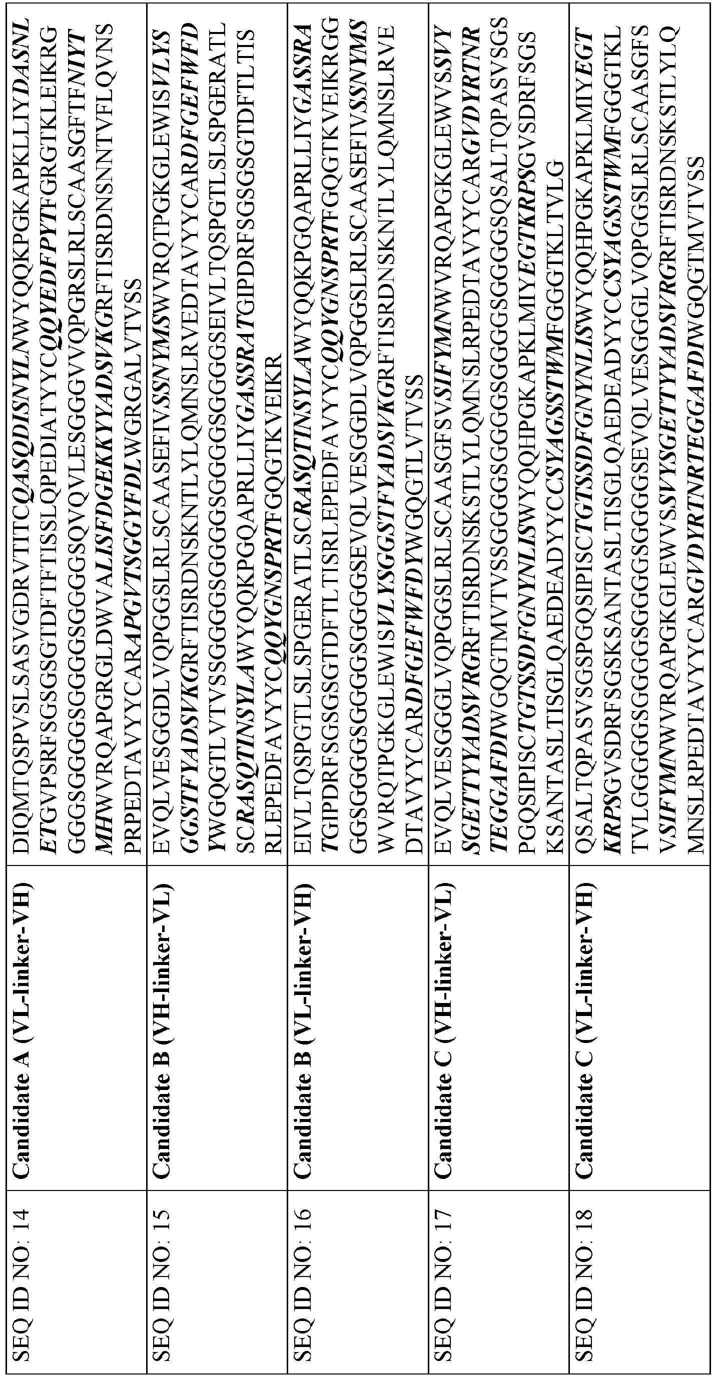

Throughout the text of this application, should there be a discrepancy between the text of the specification (*e*. *g*., Table 1) and the sequence listing, the text of the specification shall prevail.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrate the invention described above, but is not, however, intended to limit the scope of the invention in any way. Other test models known as such to the person skilled in the pertinent art can also determine the beneficial effects of the claimed invention.

### Examples

### Example 1: Identification of antibodies specific for the S protein of SARS-CoV-2

As a team, we have established an innovative pipeline for the generation of patient-derived mAbs against SARS-CoV-2, which we termed MEMUMAB (Figure 1), wherein we isolated memory B cells specific for the S protein of SARS-CoV-2 from convalescent COVID-19 patients.

### 1.1 Antigen baiting of SARS-CoV-2 specific memory B cells

Peripheral Blood Mononuclear Cell (PBMC) of SARS-CoV-2 convalescent patients were isolated by Ficoll gradient centrifugation of EDTA-blood. 1×10⁷ PBMC were incubated with recombinant biotinylated SARS-CoV-2 protein (either the whole S protein or the receptor binding domain (RBD)) at a concentration of 10 µg/ml in a volume of 200 µl MACS buffer for 25 min. After washing twice, the cells were labeled with a mastermix containing CD19-FITC, IgD-PE-CF594, IgM-BV605 & PE-Streptavidin.

Single CD19 and PE positive B-cells with negative staining for IgD and IgM (class-switched B cells) were sorted into wells of a 96-well plate using a BD FACSAria Fusion sorter. V(D)J sequences were retrieved as described¹. Hundreds of individual B cells were sequenced to decipher their variable region gene sequences of the immunoglobulin heavy and light chains. Heavy and Light V(D)J sequences that passed the manual curation for completeness and quality were used further for monoclonal antibody expression.

### Example 2: Antibody cloning and expression

Upon vectorization, the sequences identified in Example 1 were expressed as human IgG mAbs. For this purpose, clinically used lentiviral SIN vectors were used and expressed in fully traceable HEK293T producer cells. The supernatants containing recombinant mAbs were extensively tested for their biological activities. Of note, when compared with the corresponding germline antibodies, the mutated antibodies as retrieved from memory B cells have been subjected *in vivo* to excessive affinity-maturation. Thus, the MEMUMAB pipeline allowed identification of promising new mAb candidates within a short period of time and the mAbs could be stably produced and harvested from the culture supernatant for immediate analysis and/or purification.

### 2.1 Expression of monoclonal antibodies

The V(D)J sequences were codon-optimized and produced as gene strings using gene synthesis. Using cloning strategies and restriction enzymes, the synthesized sequences were incorporated into a lentiviral (LV) vector plasmid for IgG1 antibody expression. For screening of the clones, the antibodies were produced as scFv-IgG1 fusion protein first. Promising candidates were later expressed as full IgG1 antibodies for characterization. Lentiviral packing of the vector plasmid [containing the gene of interest (GOI)] was done in 293T cells using a transfection technique based on a 4 plasmid, third generation lentiviral SIN vector system (Schambach et al., Mol. Ther. 13 (2006) 391-400). 293T cells were plated at 1×10⁶ cells per well of a 6 well plate. On the following day (cells > 90 % confluent), 1 µg of vector plasmid was used to transfect the cells using a scale-down of a calcium phosphate protocol for LV vector production which was developed in-house. Medium on the transfected cells was changed after 16-18 h. Vector supernatant was harvested and sterile filtered 24 h post medium change. Vector supernatant was stored at -80°C until used for transduction of the producer cells.

In brief, the 293T mAb producer cells were plated at 30,000 cells per well in a 12 well plate. 250 µl of vectors (heavy and light chain vectors were produced separately, cells were co-transduced with equal volumes of both) added on the cells along with half the total volume of fresh D10 medium containing 1:1,000 of protamine sulfate. For all-in-one vector designs, for example scFvs, 500 µl of the vector was used. Spinoculation was done at 2,000 rpm at 37°C for 60 min. Cells were then detached and expanded in a 6 well plate with 3 ml of fresh D10 medium for antibody harvesting.

### Example 3: Antibody characterization

### 3.1 ELISA binding assays

The supernatants generated in Example 2 were used to quantify their IgG1 concentration by ELISA (Invitrogen BMS2092). Three different ELISA were performed on 1:5 diluted supernatants: Binding to the trimeric Spike (Invitrogen BMS2325), the S1 Domain (Euroimmun EI 2606-9601 G) and the RBD (Biolegend 447707). The OD₄₅₀ was determined, and if the signal was too strong for quantification, it was manually set to OD=4. The results of these ELISA experiments are shown in Fig. 2, columns 1 to 3.

### 3.2 Neutralization assays

For screening of all supernatants, a 1:5 dilution of each supernatant was measured in a surrogate neutralization assay (Euroimmun EI 2606-9601-4), in which the sample of interest competes with a recombinant ACE2 for binding to a coated RBD. The readout represents the binding of ACE2 to RBD and a decrease in signal is indicative of neutralization. The OD₄₅₀ was determined, and the neutralization strength was calculated as OD_{blank}-ODₛₐₘₚₗₑ. The results of that ELISA experiment are shown in Figure 2, column 4.

As comparators, Casirivimab & Imdevimab were retrieved from Regeneron and Bamlanivimab & Etesevimab^{2,3} were expressed similarly as described in Example 2. The results of a comparison of the neutralization capacities of the three candidates A, B, and C with those already marketed SARS-CoV-2 neutralizing mAbs are shown in Fig. 3.

Samples which showed a promising IgG1 concentration to neutralization strength ratio were selected for further characterization. The neutralization of VSV pseudoparticles carrying different variants of the SARS-CoV-2 Spike⁴ was tested by pre-incubating different concentrations of the antibodies with the pseudoparticles (96 well formate with a cfu of 100/well) for 1h at 37°C. Afterwards, Vero cells growing in a confluent layer in a 96 well plate were infected with the mixture and the infectivity was recorded by fluorescence microscopy. The number of GFP+ cells per well was counted and plotted against the antibody concentration.

The neutralization capacity of the antibodies was verified by serial dilution of the samples, mixing with SARS-CoV-2 (Wuhan isolate) and incubation for 90 min at 37°C. Afterwards, Vero cells growing in a confluent layer in a 96 well plate were infected with the mixture and overlayed with methylcellulose for plaque formation. The concentration of antibody which prevented plaque formation completely was noted as the IC₁₀₀ value.

### Example 4: Evidence for improved binding through affinity maturation

Since the variable region genes of our candidates deviate substantially from the respective germline sequence, we hypothesized that they were subjected to extensive hypermutations *in vivo,* which might be associated with affinity maturation of the antibodies. To study this, we expressed germline versions of the mAb candidates with reverted V_{H}/V_{L} mutations and compared their binding strengths in an ELISA assay with those of the original mAbs. Indeed, candidates A and B showed substantially increased binding strengths in their hypermutated form, whereas candidate C did not show a binding difference of the hypermutated and the germline version (Figure 4). Interestingly, candidate C neutralized more potently than the germline version. Additionally, it neutralized also VOCs, which exhibited resistance against the germline version (data not shown).

### Example 5: Variants of concern (VOCs) and viral escape

It has been described that certain variants of SARS-CoV-2 have acquired the ability to resist neutralization by patient sera as well as by several mAbs. While the Alpha variant (B.1.1.7) only weakly escapes neutralization, this effect is very strong for the Beta (B.1.351) and Gamma (P.1) variants, which can even re-infect convalescent patients in some cases. Furthermore, the Delta variant that was first detected in India in the beginning of 2021 (B.1.617.2) rapidly spread over the whole world and now is the dominating virus variant in most countries.

We investigated the capacity of our three candidates to neutralize the known VOCs of SARS-CoV-2 using genetically engineered VSV pseudoparticles. Indeed, our candidates proved to be potent cross-neutralizing mAbs showing similar neutralization of the Alpha, Beta, Gamma, and Delta virus variants with only minor differences in the neutralization capacity amongst the three mAb candidates (Figure 5(A)).

### 5.1 Viral escape assay

Serial dilutions of the antibody or of antibody cocktails were mixed with 5×10⁵ cfu of a recombinant VSV virus, which expresses the SARS-CoV-2 Spike instead of the VSV glycoprotein in a total volume of 1 ml. A 24 well plate with confluent Vero cells was infected with the mixtures and incubated for 4 days. Supernatant from the respective wells with highest antibody concentration which still showed infectivity (GFP+ cell layer) were used in a second round of the experiment to verify the resistance against the respective antibody as previously described⁵.

Viral RNA was isolated from the infected cell layers, cDNA was synthesized and the RBD region was amplified by PCR. The SNPs which are associated with viral escape were identified by Sanger sequencing of the PCR product. Finally, the three-dimensional location of the SNP was mapped to a published SARS-CoV-2 Spike structure (PDB 7KNB) using PyMol.

To investigate which mutations in the RBD domain of the SARS-CoV-2 spike protein might confer viral escape, we performed an enforced virus escape assay in which a large amount of recombinant VSV in which the endogenous G protein is replaced by the WT spike protein was inoculated in the presence of different mAb concentrations. This system allows for fast screening of possible escape variants as VSV has a higher mutation rate than SARS-CoV-2. We observed that while the virus rapidly escaped from the treatment with single mAbs, remarkably, all three combinations of our mAb candidates suppressed viral escape to non-detectable levels (Figure 5(B)).

### Example 6: Analysis of distinct epitopes on the RBD are bound by the MEMUMAB candidates

The three investigated candidates consist of non-related gene fragments. Therefore, it is unlikely that they recognize identical epitopes on the surface of the spike protein. Through sequencing of the viral escape mutants followed by mapping of the identified amino acid exchanges on a published S structure, we were able to collect evidence for a distinct binding mode of the three mAbs. Figure 6 illustrates that the identified positions for viral escape are located apart from each other: The residue important for neutralization by candidate A, R346, is located on the outer side of the RBD. On the very opposite side of the RBD domain, T415 is crucial for neutralization of candidate B, making it unlikely that these mAbs share the identical binding site. Candidate C leads to escape at position F486, which is close to the ACE2 binding interface. Of note, none of the identified mutations is also detected in VOCs. However, the mutation R346K is present in the variant of interest (VOI) B.1.621, which was first detected in Columbia in Jan. 2021, but is only detected sporadically.

### References:

1. Guthmiller, J.J., Dugan, H.L., Neu, K.E., Lan, L.Y. & Wilson, P.C. An Efficient Method to Generate Monoclonal Antibodies from Human B Cells. Methods Mol Biol 1904, 109-145 (2019).
2. Shi, R. et al. A human neutralizing antibody targets the receptor-binding site of SARS-CoV-2. Nature 584, 120-124 (2020).
3. Jones, B.E. et al. The neutralizing antibody, LY-CoV555, protects against SARS-CoV-2 infection in nonhuman primates. Sci Transl Med 13, eabf1906 (2021).
4. Hoffmann, M. et al. SARS-CoV-2 variants B.1.351 and P.1 escape from neutralizing antibodies. Cell 184, 2384-2393.e12 (2021).
5. Baum, A. et al. Antibody cocktail to SARS-CoV-2 spike protein prevents rapid mutational escape seen with individual antibodies. Science 369 (2020) 1014-1018.

## Claims

1. An antibody-based binding composition with specificity for the SARS-CoV-2 spike receptor-binding domain, wherein said antibody-based binding composition comprises a combination of variable domains selected from the group of
(i) a VH domain comprising at least residues 3 to 138 according to the AHo numbering system of the VH domain according to SEQ ID NO:1, and a VL domain comprising at least residues 3 to 138 according to the AHo numbering system of the VL domain according to SEQ ID NO:2;
(ii) a VH domain comprising at least residues 3 to 138 according to the AHo numbering system of the VH domain according to SEQ ID NO:3, and a VL domain comprising at least residues 3 to 138 according to the AHo numbering system of the VL domain according to SEQ ID NO:4; and
(iii) a VH domain comprising at least residues 3 to 138 according to the AHo numbering system of the VH domain according to SEQ ID NO:5, and a VL domain comprising at least residues 3 to 138 according to the AHo numbering system of the VL domain according to SEQ ID NO:6.

2. The antibody-based binding composition of claim 1, wherein said antibody-based binding composition is a full-length antibody, particularly a full-length antibody selected from the list of IgA, IgD, IgE and IgG.

3. The antibody-based composition of claim 2, wherein said full-length antibody is an IgG, particularly an IgG selected from the list of IgG1, IgG2, IgG3 and IgG4.

4. The antibody-based binding composition of claim 1, wherein said antibody-based binding composition is an antibody-based antigen-binding fragment, particularly an antigen-binding fragment selected from the list of: Fab fragment, Fab' fragment, F(ab')₂ fragment, Fv fragment, dsFv fragment, scFv fragment, dsscFv fragment and a fusion protein comprising a Fab fragment, a Fab' fragment, a F(ab')₂ fragment, an Fv fragment, a dsFv fragment, an scFv fragment and a dsscFv fragment, particularly wherein said antigen-binding fragment is an scFv fragment or a fusion protein comprising an scFv fragment, particularly an scFv-Fc fusion protein or an (scFv)₂-Fc fusion protein.

5. The antibody-based binding composition of claim 4, wherein said antibody-based antigen-binding fragment is an scFv fragment selected from the list of scFv fragments according to SEQ ID NOs: 13 to 18.

6. A multispecific antibody-based binding composition with specificity for at least two different epitopes on the SARS-CoV-2 spike receptor-binding domain, wherein said multispecific antibody-based binding composition comprises a first antibody-based binding composition, which is an antibody-based binding composition of claim 1, and at least a second antibody-based binding composition with specificity for an epitope on the SARS-CoV-2 spike receptor-binding domain, which is different from the epitope of said first antibody-based binding composition.

7. The multispecific antibody-based binding composition of claim 6, wherein said second antibody-based binding composition is an antibody-based binding composition of claim 1.

8. The multispecific antibody-based binding composition of claim 6 or 7, wherein said first and said second antibody-based binding composition are present as two separate monospecific constructs.

9. The multispecific antibody-based binding composition of claim 6 or 7, wherein said first and said second antibody-based binding composition are comprised in one multispecific construct, particularly a multispecific construct selected from the list of: diabody; scDb; tandem di-scFv, tandem tri-scFv; Fab-scFv; scFab-dsscFv; Fab-(scFv)₂; Fab₂; Fab-(Fv)₂; triabody; scDb-scFv; tandem tri-scFv, a scFab-dsscFv, and constructs comprising two antigen-based binding compositions selected from Fab fragment, Fab' fragment, F(ab')₂ fragment, Fv fragment, dsFv fragment, scFv fragment and dsscFv fragment, wherein each of said two antigen-based binding compositions is fused to the N- and/or the C-terminus of one chain of a heterodimerization domain, particularly one chain of a heterodimeric Fc domain.

10. A nucleic acid encoding the antibody-based binding composition of any one of claims 1 to 5, or the multispecific antibody-based binding composition of any one of claims 6 to 9.

11. The nucleic acid of claim 10, which is part of a lentiviral or AAV-based vector system.

12. The nucleic acid of claim 11, wherein said lentiviral or AAV-based vector system is suitable for the *in vivo* expression of the antibody-based binding composition of any one of claims 1 to 5, or of the multispecific antibody-based binding composition of any one of claims 6 to 9.

13. A pharmaceutical composition comprising the antibody-based binding composition of any one of claims 1 to 5, the multispecific antibody-based binding composition of any one of claims 6 to 9, or the nucleic acid of claim 10, and a pharmaceutically acceptable carrier.

14. The pharmaceutical composition of claim 11, wherein said pharmaceutically acceptable carrier is a carrier that is suitable for intranasal or inhalative application of said pharmaceutical composition.

15. The antibody-based binding composition of any one of claims 1 to 5, the multispecific antibody-based binding composition of any one of claims 6 to 9, the nucleic acid of any one of claims 10 to 12, or the pharmaceutical composition of claim 13 or 14, for use in the prevention or treatment of an infection with SARS-CoV-2.
